(19) Europäisches Patentamt
European Patent Office
Office européen des brevets

(11) Veröffentlichungsnummer: **0 229 963 B1**

(12) # EUROPÄISCHE PATENTSCHRIFT

(45) Veröffentlichungstag der Patentschrift: **11.09.91**

(51) Int. Cl.5: **C07D 491/04**, A61K 31/435, //(C07D491/04,307:00,221:00)

(21) Anmeldenummer: **86116982.9**

(22) Anmeldetag: **06.12.86**

Die Akte enthält technische Angaben, die nach dem Eingang der Anmeldung eingereicht wurden und die nicht in dieser Patentschrift enthalten sind.

(54) **4-Thienyl-dihydropyridine, Verfahren zur Herstellung und ihre Verwendung in Arzneimitteln.**

(30) Priorität: **18.12.85 DE 3544693**

(43) Veröffentlichungstag der Anmeldung: **29.07.87 Patentblatt 87/31**

(45) Bekanntmachung des Hinweises auf die Patenterteilung: **11.09.91 Patentblatt 91/37**

(84) Benannte Vertragsstaaten: **AT BE CH DE ES FR GB IT LI NL SE**

(56) Entgegenhaltungen:
**EP-A- 0 111 453**
**EP-A- 0 111 455**
**DE-A- 2 629 892**

(73) Patentinhaber: **BAYER AG**

**W-5090 Leverkusen 1 Bayerwerk(DE)**

(72) Erfinder: **Goldmann, Siegfried, Dr.**
**Am Osterholz 91**
**W-5600 Wuppertal 11(DE)**
Erfinder: **Bischoff, Hilmar, Dr.**
**Wilkhausstrasse 107**
**W-5600 Wuppertal 2(DE)**
Erfinder: **Puls, Walter, Dr.**
**In den Birken 75**
**W-5600 Wuppertal 1(DE)**
Erfinder: **Bender, Joachim, Dr.**
**Steinberger Weg 52**
**W-5600 Wuppertal 1(DE)**
Erfinder: **Petzinna, Dieter, Dr. Dr.**
**Baustrasse 26**
**W-4230 Wesel(DE)**
Erfinder: **Schlossmann, Klaus, Dr.**
**In der Beek 116**
**W-5600 Wuppertal 1(DE)**

EP 0 229 963 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 4-Thienyl-dihydropyridinlactone, Verfahren zu ihrer Herstellung sowie ihre Verwendung in Arzneimitteln, insbesondere in blutzuckerbeeinflussenden Arzneimitteln.

Aus EP-A-0 011 453 sind bereits Dihydropyridine, die in 1-Position unsubstituiert sind, als Zwischenprodukte bekannt. Im Gegensatz zu den N-substitutierten erfindungsgemäßen Verbindungen, die eine signifikante Senkung der Blutglukose bewirken, zeigen die bekannten N-H-Verbindungen keinen entsprechenden Effekt. Es konnte nicht erwartet werden, daß eine solche geringfügige Modifikation in der Struktur so gravierende Änderungen in der Wirkung hervorruft.

Die vorliegende Erfindung betrifft 4-Thienyl-dihydropyridine der allgemeinen Formel (I)

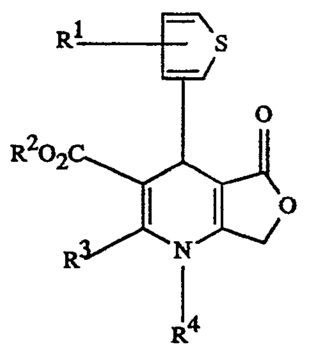

(I)

in welcher

- $R^1$ für Fluor, Chlor oder für geradkettiges oder verzweigtes Alkyl mit bis zu 4 Kohlenstoffatomen steht,
- $R^2$ für einen geradkettigen, verzweigten oder cyclischen Kohlenwasserstoffrest mit bis zu 8 Kohlenstoffatomen steht, der gegebenenfalls durch ein Sauerstoffatom unterbrochen ist und der gegebenenfalls substituiert ist durch Fluor, Chlor, Phenyl, Hydroxy, Cyano, Amino, $C_1$-$C_3$-Alkylamino, Di-$C_1$-$C_3$-alkylamino oder N-Benzyl-N-methyl-amino,
- $R^3$ für gegebenenfalls durch Hydroxy substituiertes $C_1$-$C_4$-Alkyl steht, und
- $R^4$ für einen geradkettigen, verzweigten oder cyclischen Alkylrest mit bis zu 6 Kohlenstoffatomen steht,

in Form ihrer Isomeren, Isomerengemische, Racemate und optische Antipoden, sowie ihre physiologisch unbedenklichen Salze.

Insbesondere seien solche Verbindungen der Formel (I) genannt, in denen

- $R^1$ - für Chlor oder $C_1$-$C_4$-Alkyl steht,
- $R^2$ - für einen geradkettigen oder verzweigten Alkylrest mit bis zu 6 Kohlenstoffatomen steht,
- $R^3$ - für $C_1$-$C_4$-Alkyl steht

und

- $R^4$ - für Wasserstoff oder einen Alkylrest mit bis zu 4 Kohlenstoffatomen steht,

in Form ihrer Isomeren, Isomerengemische, Racemate und optischen Antipoden sowie ihre physiologisch unbedenklichen Salze.

Die erfindungsgemäßen Stoffe können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen oder organischen Säuren. Als Beispiele seien genannt:
Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) können hergestellt werden, indem man

[A] Aldehyde der allgemeinen Formel II

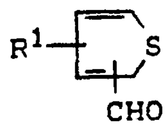

(II)

in welcher
$R^1$ die angegebene Bedeutung hat,
mit Acetessigestern der allgemeinen Formel III

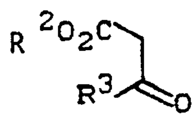

(III)

in welcher
$R^2$, $R^3$ die oben angegebene Bedeutung haben,
mit Enaminen der allgemeinen Formel IV

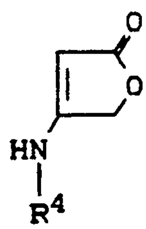

(IV)

in welcher
$R^4$ die angegebene Bedeutung hat,
in inerten Lösungsmitteln umsetzt, oder wenn man
[B] Ylidenverbindungen der allgemeinen Formel V,

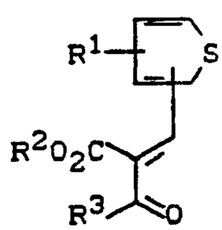

V

in welcher
$R^1$, $R^2$, $R^3$ die angegebene Bedeutung haben,
mit Enaminen der Formel IV in inerten Lösungsmitteln umsetzt,
oder wenn man
[C] Dihydropyridinlactone der allgemeinen Formel VI

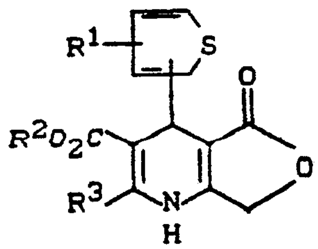

VI

in welcher

$R^1$ bis $R^3$ die angegebene Bedeutung haben,

in inerten Lösungsmitteln mit Basen behandelt und anschließend mit Verbindungen der allgemeinen Formel VII

$R^4$-X VII

in welcher

$R^4$ die angegebene Bedeutung hat und

X für Halogen, bevorzugt Chlor, Brom oder Iod oder für eine Diazogruppe oder für eine Gruppe der Formel $-O-SO_2R^5$ steht,

worin

$R^5$ die Bedeutung von $R^4$ hat oder für Phenyl oder Tolyl steht,

alkyliert.

Je nach Art der verwendeten Ausgangsstoffe lassen sich die Verfahren durch folgende Schemata verdeutlichen:

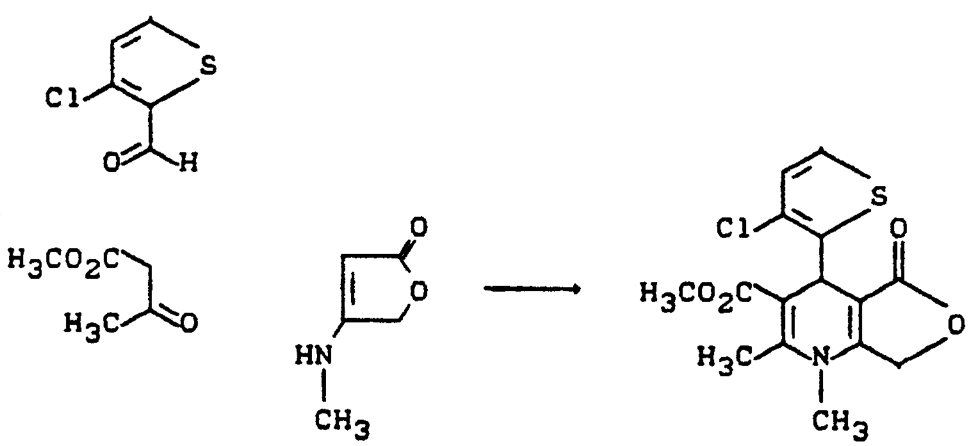

[B]

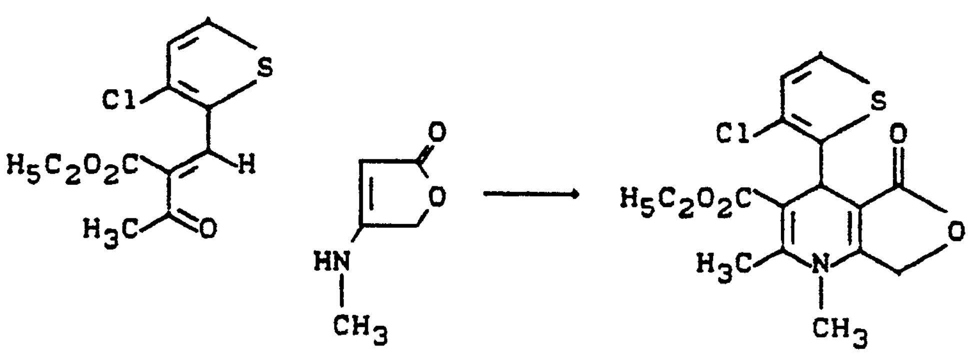

[C]

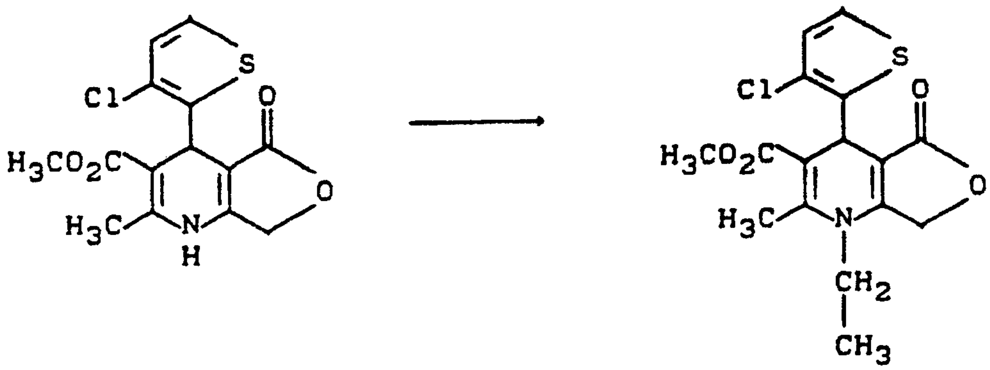

Als Lösungsmittel für Verfahren A und B kommen alle inerten organischen Lösungsmittel in Frage, die sich unter den Reaktionsbedingungen nicht verändern. Hierzu gehören bevorzugt Alkohole wie Methanol, Ethanol, Propanol, Isopropanol oder Ether wie Diethylether, Dioxan, Tetrahydrofuran, Glykolmono- oder dimethylether, Dimethylformamid, Essigsäure, Acetonitril, Pyridin, Dimethylsulfoxid oder Hexamethylphosphorsäuretriamid. Ebenso ist es möglich Gemische der genannten Lösungsmittel zu verwenden.

Als Lösungsmittel für Verfahren C kommen die üblichen inerten organischen Lösungsmittel in Frage. Hierzu gehören bevorzugt Ether wie Diethylether, Dioxan oder Tetrahydrofuran, oder Säureamide wie Dimethylformamid oder Hexamethylphosphorsäuretriamid, oder Dimethylsulfoxid oder Sulfolan, oder Kohlenwasserstoffe wie Benzol, Xylol, Toluol, Hexan oder Erdölfraktionen.

Als Basen (Verfahren C) können die zur Deprotonierung üblichen Basen verwendet werden. Hierzu gehören bevorzugt Alkalihydride wie Natriumhydrid oder Kaliumhydrid, oder Alkaliamide wie Natriumamid, Kaliumdiethylamid oder Lithiumdiisopropylamid, oder Alkalihydroxide wie Kaliumhydroxid oder Natriumhydroxid, Alkalialkoholate wie Kalium-tert.-butanolat, Kaliummethylat oder Kaliumethylat oder Metallorganyle wie Phenyllithium, n-Butyllithium oder tert.-Butyllithium.

Die Reaktionstemperaturen bei Verfahren A und B können in einem größeren Bereich variiert werden. Im allgemeinen arbeitet man in einem Bereich von 10° C bis 200° C, bevorzugt von 20° C bis 150° C.

Die Umsetzung des Dihydropyridinlactons VI mit Base (Verfahren C) erfolgt im allgemeinen in einem Temperaturbereich von -80° C bis +200° C, bevorzugt in einem Temperaturbereich von -20° C bis +180° C, besonders bevorzugt von 20° C bis zur Siedetemperatur des verwendeten Lösungsmittels.

Alle Verfahren können bei normalen, erhöhtem oder erniedrigtem Druck durchgeführt werden. Im allgemeinen arbeitet man bei Normaldruck.

Die als Ausgangsstoffe verwendeten Aldehyde der Formel II sind bekannt oder können nach bekannten Methoden hergestellt werden (vgl. US-Patent 2.601.479; US-Patent 2.853.493; J. prakt. Chem. 1964, 64).

Die als Ausgangsstoffe verwendeten Acetessigester der Formel III sind bekannt oder können nach bekannten Methoden hergestellt werden (vgl. D. Borrmann, Houben-Weyl "Methoden der organischen Chemie" Band VII 14, 230 ff ).

Die als Ausgangsstoffe verwendeten Enamine der Formel IV sind bekannt oder können nach bekannten Methoden hergestellt werden (vgl. EP 123 095).

Die als Ausgangsstoffe verwendeten Ylidenverbindungen der Formel V sind bekannt oder können nach bekannten Methoden hergestellt werden (vgl. G. Jones, "The Knoevenagel Condensation", Organic Reactions XV, 204 (1967)).

Die als Ausgangsstoffe verwendeten Dihydropyridinlactone der Formel VI sind teilweise neu. Sie können jedoch nach bekannten Methoden hergestellt werden (DOS 32.05.399).

Die erfindungsgemäßen Verbindungen der Formel I zeigen ein wertvolles pharmakologisches Wirkspektrum. Bei nur geringer Kreislaufwirkung senken sie den Blutzucker und können so zur Behandlung von Diabetes eingesetzt werden.

Die blutglucosesenkende Wirkung der zu untersuchenden Substanzen wurde an männlichen Wistar-Ratten mit einem Gewicht zwischen 140 und 190 g geprüft. Die Ratten wurden zu diesem Zweck 18 h vor der Applikation der Substanzen gewogen, in Gruppen zu 6 Tieren eingestellt und nüchtern gesetzt. Die zu untersuchenden Substanzen wurden direkt vor der Applikation in wäßriger 0,75 %iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde.

Die Blutentnahme erfolgte bei jeder Ratte 30, 60 und 120 min nach der Applikation aus dem retroorbitalen Venenplexus. Jeweils 30 µl Blut wurden mit einem automatischen Dilutor entnommen und mit 0,3 ml Uranylacetat (0,16 %) enteiweißt. Nach der Zentrifugation wurde die Glucose im Überstand nach der Glucoseoxidase-Methode mit 4-Amino-phenazon als Farbreagenz an einem Gemsaec Fastanalyzer photometrisch bestimmt. Die Auswertung der Ergebnisse erfolgte mit dem t-Test nach Student, als Signifikanzgrenze wurde $p<0,05$ gewählt.

Substanzen, die bei Ratten zu einem Zeitpunkt eine signifikante Reduktion der Blutglucosekonzentration um mindestens 10 % bewirkten, verglichen mit der Kontrollgruppe, die nur Traganthsuspension erhielt, wurden als wirksam angegeben.

Die nachfolgende Tabelle 1 enthält die gefundenen Veränderungen der Blutglucosekonzentrationen in Prozent der Kontrolle.

Tabelle 1

| Substanz (Patentbeispiel Nr.) | Abnahme der Blutglucosekonzentration in % der Kontrolle 10 mg/kg p.o. |
|---|---|
| 2 | 21 |
| 5 | 25 |
| 6 | 13 |

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen,(g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenden Überzügen und Hüllen versehen sein und auch so zusammengesetzt sein, daß sie den oder die Wirkstoffe nur oder bevorzugt in einem bestimmten Teil des Intenstinaltraktes, gegebenenfalls verzögert abgeben, wobei als Einbettungsmassen, z.B. Polymersubstanzen und Wachse

verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsmittel sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorligenden Erfindung gehört auch die Verwendung der Verbindungen der Formel I und/oder deren Salze sowie von pharmazeutischen Zubereitungen, die die Verbindungen der Formel I und/oder deren Salze enthalten, in der Human- und Veterinärmedizin zur Verhütung, Verbesserung und/oder Heilung der oben aufgeführten Erkrankungen.

Die Wirkstoffe oder die pharmazeutischen Zubereitungen können oral, parenteral, intraperitoneal und/oder rectal, vorzugsweise oral sowie parenteral appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 200 mg/kg, vorzugsweise 0,1 bis 50 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den vorgenannten Dosierungen abzuweichen und zwar in Abhängigkeit von der Art und dem Körpergewicht des zu behandelnden Objektes,der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel 1

4-(3-Chlor-2-thienyl)-1-ethyl-2-methyl-5-oxo-1.4.5.7-tetrahydrofuro[3.4-b]pyridin-3-carbonsäureisopropylester

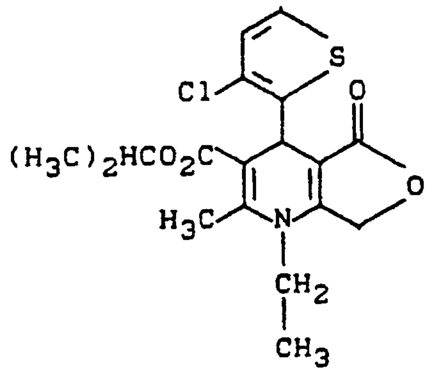

10 mmol 4-(3-Chlor-2-thienyl)-2-methyl-5-oxo-1.4.5.7-tetrahydrofuro[3.4-b]pyridin-3-carbonsäureisopropylester werden in 25 ml DMF gelöst mit NaH deprotoniert und mit 20 mmol Ethyliodid versetzt. Nach 1 h Rühren bei Raumtemperatur wird eingeengt, mit Wasser versetzt, abgesaugt und aus Methanol umkristallisiert.
Ausbeute: 75 % der Theorie
Schmelzpunkt: 76-80 °C

Beispiel 2

4-(2-Chlor-3-thienyl)-1-ethyl-2-methyl-5-oxo-1.4.5.7-tetrahydrofuro[3.4-b]pyridin-3-carbonsäureisopropylester

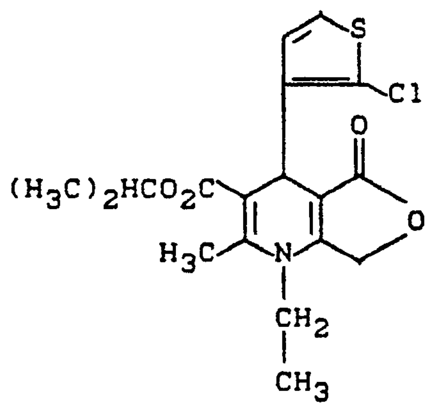

Ausbeute: 45 % der Theorie
Schmelzpunkt: 140-5 °C

Beispiel 3

1-Ethyl-2-methyl-4-(3-methyl-2-thienyl)-5-oxo-1.4.5.7-tetrahydrofuro[3.4-b]pyridin-3-carbonsäureisopropylester

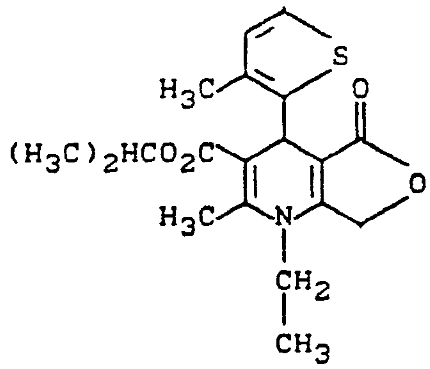

Ausbeute: 40 % der Theorie
Schmelzpunkt: 88-93° C

Beispiel 4

1,2-Dimethyl-4-(3-methyl-2-thienyl)-5-oxo-1,4,5,7-tetrahydrofuro[3.4-b]pyridin-3-carbonsäureisopropylester

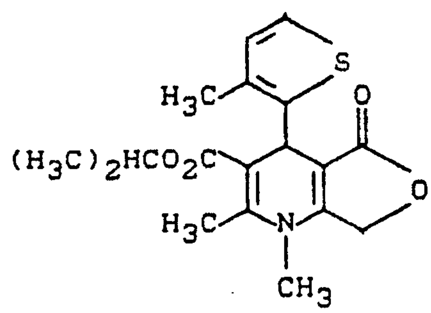

Ausbeute: 60 % der Theorie
Schmelzpunkt: 170-4° C

## Patentansprüche

**Patentansprüche für folgende Vertragsstaaten: AT, BE, CH, DE, FR, GB, IT, LI, NL, SE**

**1.** 4-Thienyl-dihydropyridine der allgemeinen Formel I

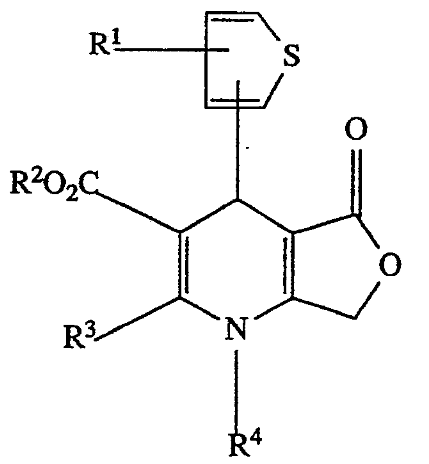

(I)

in welcher

$R^1$ für Fluor, Chlor oder für geradkettiges oder verzweigtes Alkyl mit bis zu 4 Kohlenstoffatomen steht,

$R^2$ für einen geradkettigen, verzweigten oder cyclischen Kohlenwasserstoffrest mit bis zu 8 Kohlenstoffatomen steht, der gegebenenfalls durch ein Sauerstoffatom unterbrochen ist und der gegebenenfalls substituiert ist durch Fluor, Chlor, Phenyl, Hydroxy, Cyano, Amino, $C_1$-$C_3$-Alkylamino, Di-$C_1$-$C_3$-alkylamino oder N-Benzyl-N-methyl-amino,

$R^3$ für gegebenenfalls durch Hydroxy substituiertes $C_1$-$C_4$-Alkyl steht,

und

und

$R^4$ für einen geradkettigen, verzweigten oder cyclischen Alkylrest mit bis zu 6 Kohlenstoffatomen steht,

in Form ihrer Isomeren, Isomerengemische, Racemate und optische Antipoden, sowie ihre physiologisch unbedenklichen Salze.

**2.** 4-Thienyl-dihydropyridine der allgemeinen Formel (I) in Anspruch 1, in welcher

$R^1$ für Chlor oder $C_1$-$C_4$-Alkyl steht,

$R^2$ für einen geradkettigen oder verzweigten Alkylrest mit bis zu 6 Kohlenstoffatomen steht, der gegebenenfalls durch ein Sauerstoffatom in der Kette unterbrochen ist,

$R^3$ für $C_1$-$C_4$-Alkyl steht

und

$R^4$ für Wasserstoff oder einen Alkylrest mit bis zu 4 Kohlenstoffatomen steht,

in Form ihrer Isomeren, Isomerengemische, Racemate und optischen Antipoden sowie ihre physiologisch unbedenklichen Salze.

**3.** 4-Thienyl-dihydropyridine gemäß Ansprüchen 1 und 2 zur Bekämpfung von Erkrankungen.

**4.** Arzneimittel enthaltend als Wirkstoff ein 4-Thienyl-dihydropyridin gemäß Ansprüchen 1 bis 3.

**5.** Verfahren zur Herstellung von 4-Thienyl-dihydropyridinen der allgemeinen Formel (I)

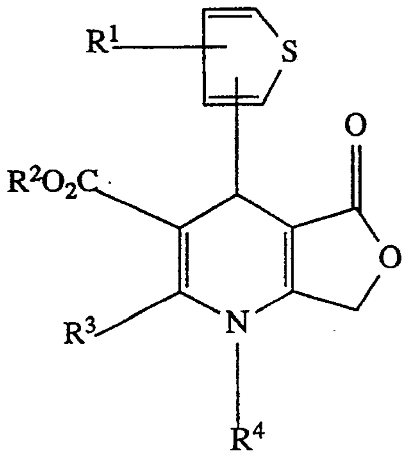

(I)

in welcher

$R^1$ für Fluor, Chlor oder für geradkettiges oder verzweigtes Alkyl mit bis zu 4 Kohlenstoffatomen steht,

$R^2$ für einen geradkettigen, verzweigten oder cyclischen Kohlenwasserstoffrest mit bis zu 8 Kohlenstoffatomen steht, der gegebenenfalls durch ein Sauerstoffatom unterbrochen ist und der gegebenenfalls substituiert ist durch Fluor, Chlor, Phenyl, Hydroxy, Cyano, Amino, $C_1$-$C_3$-Alkylamino, Di-$C_1$-$C_3$-alkylamino oder N-Benzyl-N-methyl-amino,

$R^3$ für gegebenenfalls durch Hydroxy substituiertes $C_1$-$C_4$-Alkyl steht,

und

$R^4$ für einen geradkettigen, verzweigten oder cyclischen Alkylrest mit bis zu 6 Kohlenstoffatomen steht,

in Form ihrer Isomeren, Isomerengemische, Racemate und optische Antipoden, sowie ihre physiologisch unbedenklichen Salze, dadurch gekennzeichnet, daß man

[A] Aldehyde der allgemeinen Formel II

[A] Aldehyde der allgemeinen Formel II

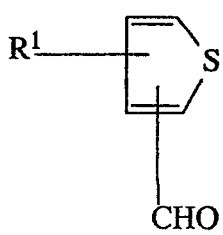

(II)

in welcher

$R^1$ die angegebene Bedeutung hat,

mit Acetessigestern der allgemeinen Formel III

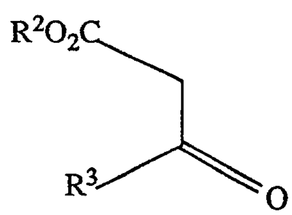

(III)

in welcher

$R^2$,$R^3$ die oben angegebene Bedeutung haben,

mit Enaminen der allgemeinen Formel IV

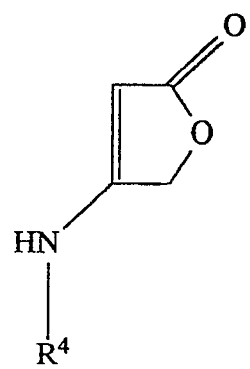

(IV)

in welcher

$R^4$ die angegebene Bedeutung hat,

in inerten Lösungsmitteln umsetzt, oder daß man

[B] Ylidenverbindungen der allgemeinen Formel V,

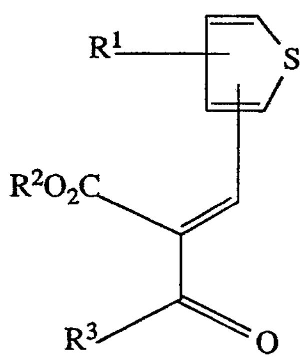

(V)

in welcher

$R^1$,$R^2$,$R^3$ die angegebene Bedeutung haben,

mit Enaminen der Formel IV in inerten Lösungsmitteln umsetzt,

oder wenn man

[C] Dihydropyidinlactone der allgemeinen Formel VI

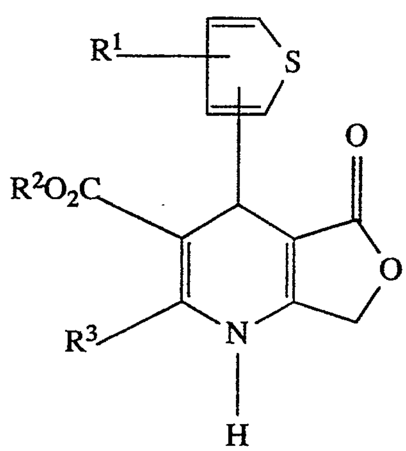

(VI)

in welcher

$R^1$ bis $R^3$ die angegebene Bedeutung haben,

in inerten Lösungsmitteln mit Basen behandelt und anschließend mit Verbindungen der allgemeinen Formel VII

$R^4$—X (VII)

in welcher

$R^4$ die angegebene Bedeutung hat und

X für Halogen, bevorzugt Chlor, Brom oder Iod oder für eine Diazogruppe oder für eine Gruppe der Formel

—O—$SO_2R^5$ steht,

worin

$R^5$ die Bedeutung von $R^4$ hat oder für Phenyl oder Tolyl steht,

alkyliert.

6. Verfahren gemäß Anspruch 5, dadurch gekennzeichnet, daß die Umsetzung gemäß Verfahrensvariante A und B bei Temperaturen von 10 bis 200 °C erfolgt.

7. Verfahren gemäß Anspruch 5, dadurch gekennzeichnet, daß die Umsetzung gemäß Verfahrensvariante C bei Temperaturen von -80 bis 200 °C erfolgt.

8. Verfahren zur Herstellung der Arzneimittel gemäß Anspruch 4, dadurch gekennzeichnet, daß man den Wirkstoff mit üblichen Hilfs- und Trägerstoffen in bekannter Weise mischt.

9. Verwendung von 4-Thienyl-dihydropyridinen gemäß Ansprüchen 1 und 2 zur Herstellung von Arzneimitteln zur Behandlung von Diabetes.

**Patentansprüche für folgenden Vertragsstaat: ES**

1. Verfahren zur Herstellung von 4-Thienyl-dihydropyridinen der allgemeinen Formel (I)

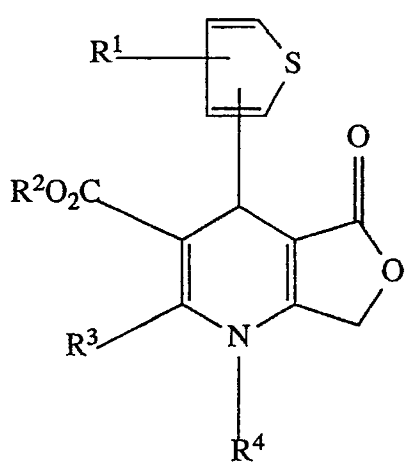

(I)

in welcher

$R^1$ für Fluor, Chlor oder für geradkettiges oder verzweigtes Alkyl mit bis zu 4 Kohlenstoffatomen steht,

$R^2$ für einen geradkettigen, verzweigten oder cyclischen Kohlenwasserstoffrest mit bis zu 8 Kohlenstoffatomen steht, der gegebenenfalls durch ein Sauerstoffatom unterbrochen ist und der gegebenenfalls substituiert ist durch Fluor, Chlor, Phenyl, Hydroxy, Cyano, Amino, $C_1$-$C_3$-Alkylamino, Di-$C_1$-$C_3$-alkylamino oder N-Benzyl-N-methyl-amino,

$R^3$ für gegebenenfalls durch Hydroxy substituiertes $C_1$-$C_4$-Alkyl steht,

und

$R^4$ für einen geradkettigen, verzweigten oder cyclischen Alkylrest mit bis zu 6 Kohlenstoffatomen steht,

in Form ihrer Isomeren, Isomerengemische, Racemate und optische Antipoden, sowie ihre physiologisch unbedenklichen Salze, dadurch gekennzeichnet, daß man

[A] Aldehyde der allgemeinen Formel II

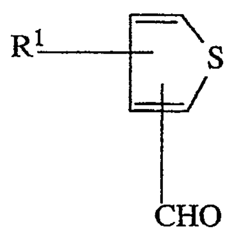 (II)

in welcher

$R^1$ die angegebene Bedeutung hat,

mit Acetessigestern der allgemeinen Formel III

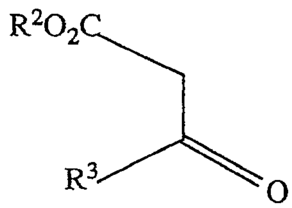 (III)

in welcher

$R^2$,$R^3$ die oben angegebene Bedeutung haben,

mit Enaminen der allgemeinen Formel IV

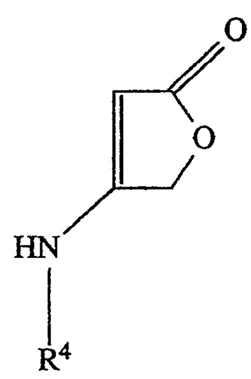 (IV)

in welcher

$R^4$ die angegebene Bedeutung hat,

in inerten Lösungsmitteln umsetzt, oder daß man

[B] Ylidenverbindungen der allgemeinen Formel V,

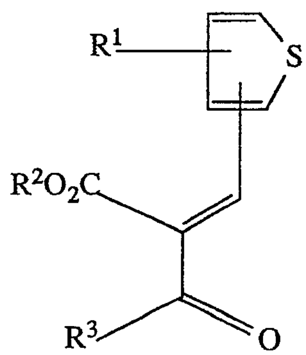

(V)

in welcher

$R^1$,$R^2$,$R^3$ die angegebene Bedeutung haben,

mit Enaminen der Formel IV in inerten Lösungsmitteln umsetzt,

oder wenn man

[C] Dihydropyidinlactone der allgemeinen Formel VI

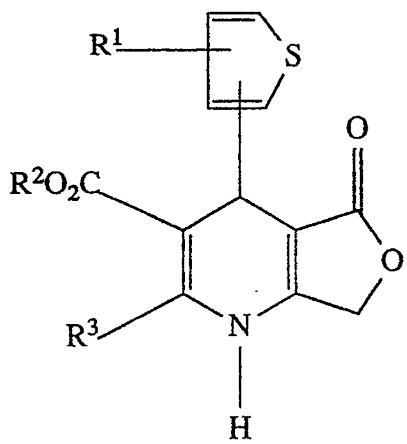

(VI)

in welcher

$R^1$ bis $R^3$ die angegebene Bedeutung haben,

in inerten Lösungsmitteln mit Basen behandelt und anschließend mit Verbindungen der allgemeinen Formel VII

$R^4$—X (VII)

in welcher

$R^4$ die angegebene Bedeutung hat und

X für Halogen, bevorzugt Chlor, Brom oder Iod oder für eine Diazogruppe oder für eine Gruppe der Formel

—O—$SO_2R^5$ steht,

worin

$R^5$ die Bedeutung von $R^4$ hat oder für Phenyl oder Tolyl steht,

alkyliert.

**2.** Verfahren gemäß Anspruch 1, dadurch gekennzeichnet, daß die Umsetzung gemäß Verfahrensvariante A und B bei Temperaturen von 10 bis 200 °C erfolgt.

3. Verfahren gemäß Anspruch 1, dadurch gekennzeichnet, daß die Umsetzung gemäß Verfahrensvariante C bei Temperaturen von -80 bis 200 °C erfolgt.

4. Verfahren zur Herstellung von Arzneimitteln dadurch gekennzeichnet, daß man man mindestens ein 4-Thienyl-dihydropyridin gemäß Formel I in Anspruch 1 mit üblichen Hilfs- und Trägerstoffen mischt.

**Claims**

**Claims for the following Contracting States: AT, BE, CH, DE, FR, GB, IT, LI, NL, SE**

1. 4-Thienyl-dihydropyridines of the general formula I

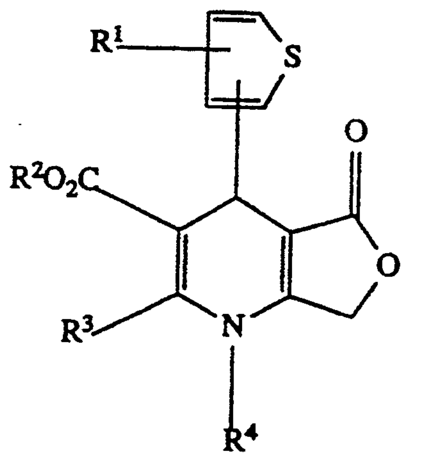

(I)

in which

$R^1$ represents fluorine or chlorine, or represents straight-chain or branched alkyl with up to 4 carbon atoms,

$R^2$ represents a straight-chain, branched or cyclic hydrocarbon radical which has up to 8 carbon atoms, is optionally interrupted by an oxygen atom and is optionally substituted by fluorine, chlorine, phenyl, hydroxyl, cyano, amino, $C_1$-$C_3$-alkylamino, di-$C_1$-$C_3$-alkylamino or N-benzyl-N-methyl-amino,

$R^3$ represents $C_1$-$C_4$-alkyl which is optionally substituted by hydroxyl and

$R^4$ represents a straight-chain, branched or cyclic alkyl radical with up to 6 carbon atoms,

in the form of their isomers, isomer mixtures, racemates and optical antipodes, and their physiologically acceptable salts.

2. 4-Thienyl-dihydropyridines of the general formula (I) in Claim 1, in which

$R^1$ represents chlorine or $C_1$-$C_4$-alkyl,

$R^2$ represents a straight-chain or branched alkyl radical which has up to 6 carbon atoms and is optionally interrupted by an oxygen atom in the chain,

$R^3$ represents $C_1$-$C_4$-alkyl and

$R^4$ represents hydrogen or an alkyl radical with up to 4 carbon atoms,

in the form of their isomers, isomer mixtures, racemates and optical antipodes, and their physiologically acceptable salts.

3. 4-Thienyl-dihydropyridines according to Claims 1 and 2 for combating diseases.

4. Medicaments containing a 4-thienyl-dihydropyridine according to Claims 1 to 3 as the active compound.

5. Process for the preparation of 4-thienyl-dihydropyridines of the general formula (I)

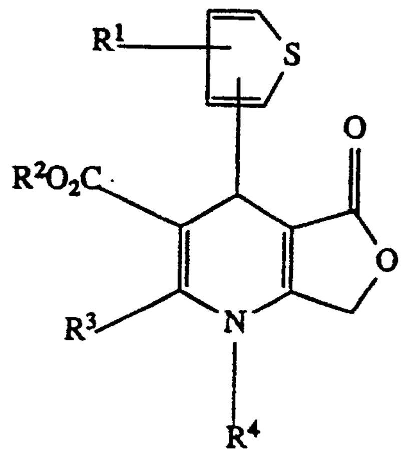

(I)

in which

- $R^1$ represents fluorine or chlorine, or represents straight-chain or branched alkyl with up to 4 carbon atoms,
- $R^2$ represents a straight-chain, branched or cyclic hydrocarbon radical which has up to 8 carbon atoms, is optionally interrupted by an oxygen atom and is optionally substituted by fluorine, chlorine, phenyl, hydroxyl, cyano, amino, $C_1$-$C_3$-alkylamino, di-$C_1$-$C_3$-alkylamino or N-benzyl-N-methyl-amino,
- $R^3$ represents $C_1$-$C_4$-alkyl which is optionally substituted by hydroxyl and
- $R^4$ represents a straight-chain, branched or cyclic alkyl radical with up to 6 carbon atoms,

in the form of their isomers, isomer mixtures, racemates and optical antipodes and their physiologically acceptable salts, characterised in that

[A] aldehydes of the general formula II

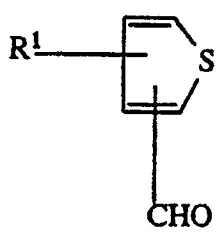

(II)

in which

$R^1$ has the meaning given,

are reacted with acetoacetic acid esters of the general formula III

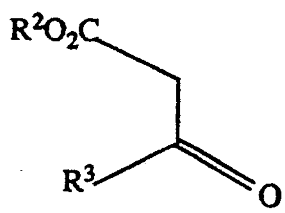

(III)

in which

$R^2$ and $R^3$ have the abovementioned meaning,

and with enamines of the general formula IV

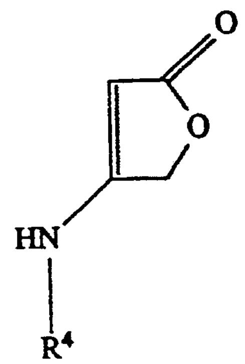

(IV)

in which

$R^4$ has the meaning given,

in inert solvents, or in that

[B] ylidene compounds of the general formula V

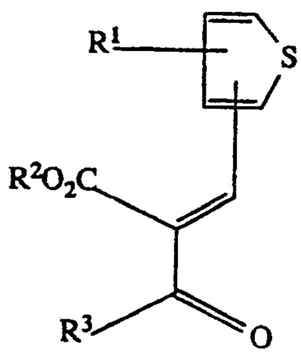

(V)

in which

$R^1$, $R^2$ and $R^3$ have the meaning given,

are reacted with enamines of the formula IV in inert solvents, or in that

[C] dihydropyridinelactones of the general formula VI

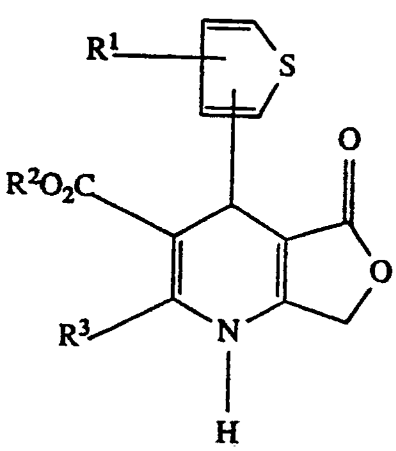

(VI)

in which

$R^1$ to $R^3$ have the meaning given,

are treated with bases in inert solvents and then alkylated with compounds of the general formula VII

$R^4$—X (VII)

in which

$R^4$ has the meaning given and

X represents halogen, preferably chlorine, bromine or iodine, or represents a diazo group, or represents a group of the formula

$-O-SO_2R^5$

wherein

$R^5$ has the meaning of $R^4$ or represents phenyl or tolyl.

6. Process according to Claim 5, characterised in that the reaction in process variants A and B is carried out at temperatures of 10 to 200° C.

7. Process according to Claim 5, characterised in that the reaction in process variant C is carried out at temperatures of -80 to 200° C.

8. Process for the preparation of the medicaments according to Claim 4, characterised in that the active compound is mixed with customary auxiliaries and excipients in a known manner.

9. Use of 4-thienyl-dihydropyridines according to Claims 1 and 2 for the preparation of medicaments for the treatment of diabetes.

**Claims for the following Contracting State: ES**

1. Process for the preparation of 4-thienyl-dihydropyridines of the general formula (I)

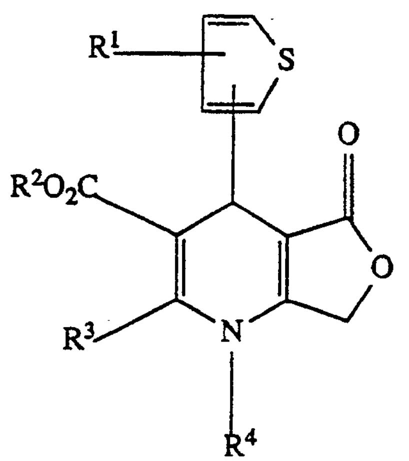

(I)

in which

$R^1$ represents fluorine or chlorine, or represents straight-chain or branched alkyl with up to 4 carbon atoms,

$R^2$ represents a straight-chain, branched or cyclic hydrocarbon radical which has up to 8 carbon atoms, is optionally interrupted by an oxygen atom and is optionally substituted by fluorine, chlorine, phenyl, hydroxyl, cyano, amino, $C_1$-$C_3$-alkylamino, di-$C_1$-$C_3$-alkylamino or N-benzyl-N-methyl-amino,

$R^3$ represents $C_1$-$C_4$-alkyl which is optionally substituted by hydroxyl and

$R^4$ represents a straight-chain, branched or cyclic alkyl radical with up to 6 carbon atoms,

in the form of their isomers, isomer mixtures, racemates and optical antipodes and their physiologically acceptable salts, characterised in that

[A] aldehydes of the general formula II

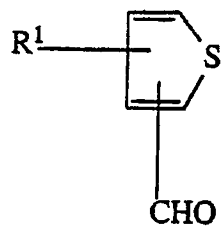

(II)

in which

$R^1$ has the meaning given,

are reacted with acetoacetic acid esters of the general formula III

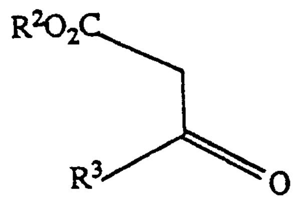

(III)

in which

$R^2$ and $R^3$ have the abovementioned meaning,

and with enamines of the general formula IV

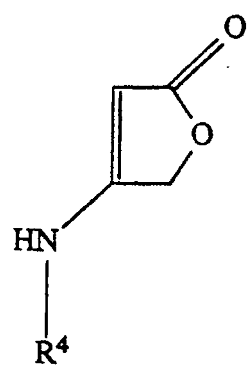

(IV)

in which

$R^4$ has the meaning given,

in inert solvents, or in that

[B] ylidene compounds of the general formula V

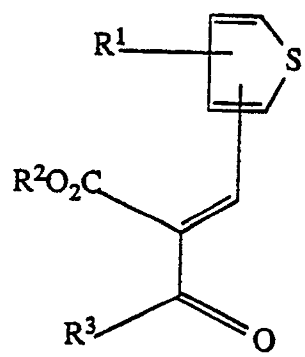

(V)

in which

$R^1$, $R^2$ and $R^3$ have the meaning given,

are reacted with enamines of the formula IV in inert solvents, or in that

[C] dihydropyridinelactones of the general formula VI

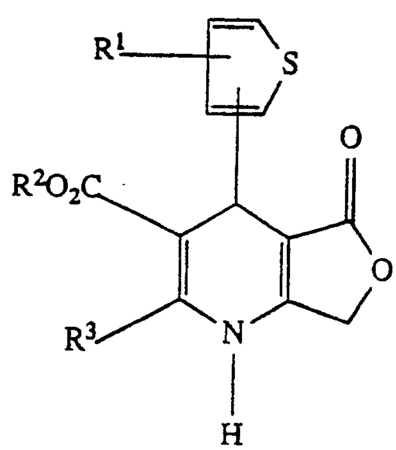

(VI)

in which

$R^1$ to $R^3$ have the meaning given,

are treated with bases in inert solvents and then alkylated with compounds of the general formula VII

$R^4$—X (VII)

in which

$R^4$ has the meaning given and

X represents halogen, preferably chlorine, bromine or iodine, or represents a diazo group, or represents a group of the formula

$—O—SO_2R^5$

wherein

$R^5$ has the meaning of $R^4$ or represents phenyl or tolyl.

**2.** Process according to Claim 1, characterised in that the reaction in process variant A and B is carried out at temperatures of 10 to 200 °C.

**3.** Process according to Claim 1, characterised in that the reaction in process variant C is carried out at temperatures of -80 to 200 °C.

4. Process for the preparation of medicaments, characterised in that at least one 4-thienyl-dihydropyridine according to formula I in Claim 1 is mixed with customary auxiliaries and excipients.

**Revendications**

**Revendications pour les Etats contractants suivants: AT, BE, CH, DE, FR, GB, IT, LI, NL, SE**

1. 4-thiényl-dihydropyridines de formule générale I

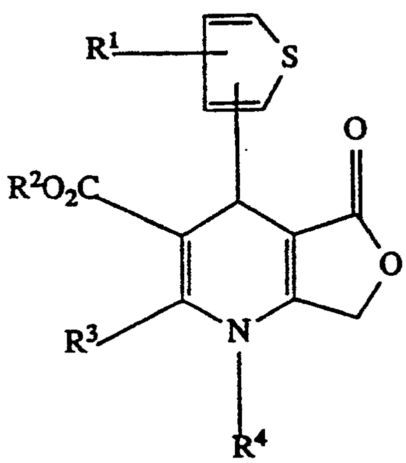

(I)

dans laquelle

$R^1$ représente un atome de fluor, un atome de chlore ou un groupe alkyle à chaîne droite ou ramifiée contenant jusqu'à 4 atomes de carbone,

$R^2$ représente un radical d'hydrocarbure à chaîne droite, à chaîne ramifiée ou cyclique, contenant jusqu'à 8 atomes de carbone, qui peut être éventuellement interrompu par un atome d'oxygène et qui est éventuellement substitué par un atome de fluor, un atome de chlore, un groupe phényle, un groupe hydroxyle, un groupe cyano, un groupe amino, un groupe alkyl(en $C_1$-$C_3$)amino, un groupe dialkyl(en $C_1$-$C_3$)amino ou un groupe N-benzyl-N-méthyl-amino,

$R^3$ représente un groupe alkyle en $C_1$-$C_4$ éventuellement substitué par un groupe hydroxyle, et

$R^4$ représente un radical alkyle à chaîne droite, à chaîne ramifiée ou cyclique contenant jusqu'à 6 atomes de carbone,

sous forme de leurs isomères, de leurs mélanges d'isomères, de leurs racémates et de leurs antipodes optiques, ainsi que leurs sels physiologiquement acceptables.

2. 4-thiényl-dihydropyridines répondant à la formule générale (I), selon la revendication 1, dans lesquelles

$R^1$ représente un atome de chlore ou un groupe alkyle en $C_1$-$C_4$,

$R^2$ représente un radical alkyle à chaîne droite ou à chaîne ramifiée contenant jusqu'à 6 atomes de carbone, qui peut éventuellement être interrompu dans la chaîne par un atome d'oxygène,

$R^3$ représente un groupe alkyle en $C_1$-$C_4$, et

$R^4$ représente un atome d'hydrogène ou un radical alkyle contenant jusqu'à 4 atomes de carbone,

sous forme de leurs isomères, de leurs mélanges d'isomères, de leurs racémates et de leurs antipodes optiques, ainsi que leurs sels physiologiquement acceptables.

3. 4-thiényl-dihydropyridines selon les revendications 1 et 2 pour lutter contre des maladies.

4. Médicament contenant, comme substance active, une 4-thiényl-dihydropyridine selon les revendications 1 à 3.

5. Procédé pour la préparation des 4-thiényl-dihydropyridines répondant à la formule générale (I)

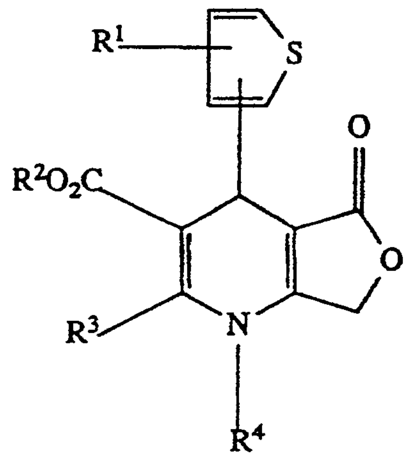

(I)

dans laquelle

R¹ représente un atome de fluor, un atome de chlcre ou un groupe alkyle à chaîne droite ou ramifiée contenant jusqu'à 4 atomes de carbone,

$R^2$ représente un radical d'hydrocarbure à chaîne droite, à chaîne ramifiée ou cyclique, contenant jusqu'à 8 atomes de carbone, qui peut être éventuellement interrompu par un atome d'oxygène et qui est éventuellement substitué par un atome de fluor, un atome de chlore, un groupe phényle, un groupe hydroxyle, un groupe cyano, un groupe amino, un groupe alkyl(en $C_1$-$C_3$)amino, un groupe dialkyl(en $C_1$-$C_3$)amino ou un groupe N-benzyl-N-méthyl-amino,

$R^3$ représente un groupe alkyle en $C_1$-$C_4$ éventuellement substitué par un groupe hydroxyle, et

$R^4$ représente un radical alkyle à chaîne droite, à chaîne ramifiée ou cyclique contenant jusqu'à 6 atomes de carbone,

sous forme de leurs isomères, de leurs mélanges d'isomères, de leurs racémates et de leurs antipodes optiques, ainsi que de leurs sels physiologiquement acceptables, **caractérisé en ce qu'**on fait réagir

[A] des aldéhydes de formule générale II

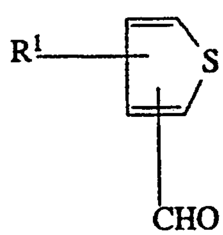

(II)

dans laquelle

$R^1$ a la signification mentionnée,

avec des esters acétiques de formule générale III

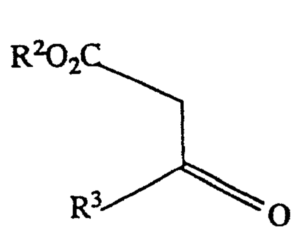

(III)

dans laquelle

$R^2$, $R^3$ ont la signification mentionnée ci-dessus,

avec des énamines de formule générale IV

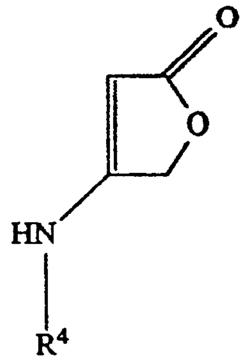

(IV)

dans laquelle

$R^4$ a la signification mentionnée,

dans des solvants inertes, ou lorsqu'on fait réagir

[B] des composés ylidène de formule générale V

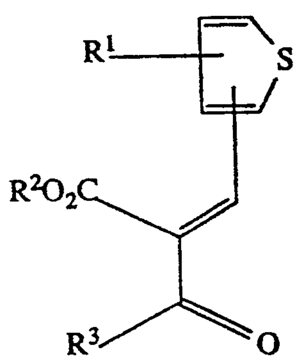

(V)

dans laquelle

$R^1$, $R^2$, $R^3$ ont la signification mentionnée,

avec des énamines de formule IV dans des solvants inertes

ou encore lorsqu'on traite avec des bases

[C] des dihydropyridine-lactones répondant à la formule générale VI

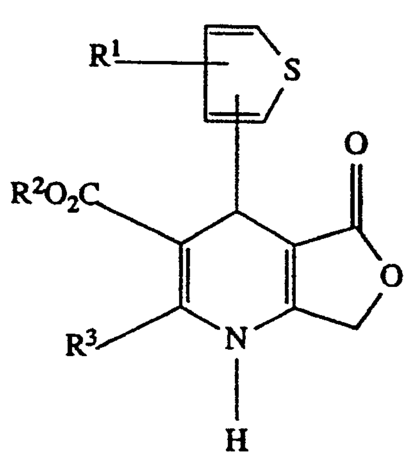

(VI)

dans laquelle

$R^1$ à $R^3$ ont les significations mentionnées,

dans des solvants inertes et ensuite, on alkyle avec des composés de formule générale VII

$R^4$-X (VII)

dans laquelle

$R^4$ a la signification mentionnée et

X représente un atome d'halogène, de préférence un atome de chlore, un atome de brome ou un atome d'iode, ou encore un groupe diazoïque ou un groupe de formule $-O-SO_2R^5$

où

$R^5$ a la signification mentionnée pour $R^4$ ou bien représente un groupe phényle ou un groupe tolyle.

**6.** Procédé selon la revendication 5, **caractérisé en ce qu'**on effectue la réaction conformément aux variantes de procédé A et B, à des températures de 10 à 200° C.

**7.** Procédé selon la revendication 5, **caractérisé en ce qu'**on effectue la réaction conformément à la variante de procédé C, à des températures de -80 à 200° C.

**8.** Procédé pour la préparation des médicaments selon la revendication 4, **caractérisé en ce qu'**on mélange la substance active avec les substances de support et auxiliaires habituelles, de manière connue.

**9.** Utilisation de 4-thiényl-dihydropyridines selon les revendications 1 et 2 pour la préparation de médicaments destinés au traitement du diabète.

**Revendications pour l'Etat contractant suivant: ES**

**1.** Procédé pour la préparation de 4-thiényl-dihydropyridines de formule générale (I)

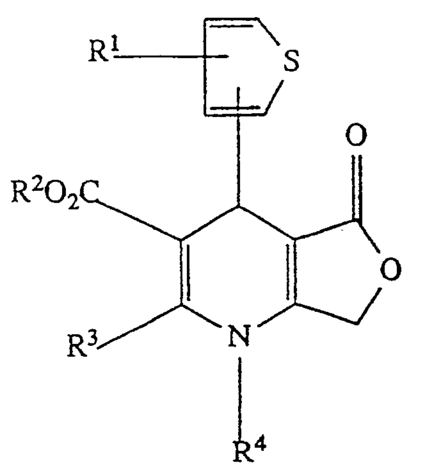

(I)

dans laquelle

$R^1$ représente un atome de fluor, un atome de chlore ou un groupe alkyle à chaîne droite ou ramifiée contenant jusqu'à 4 atomes de carbone,

$R^2$ représente un radical d'hydrocarbure à chaîne droite, à chaîne ramifiée ou cyclique, contenant jusqu'à 8 atomes de carbone, qui peut être éventuellement interrompu par un atome d'oxygène et qui est éventuellement substitué par un atome de fluor, un atome de chlore, un groupe phényle, un groupe hydroxyle, un groupe cyano, un groupe amino, un groupe alkyl(en $C_1$-$C_3$)amino, un groupe dialkyl(en $C_1$-$C_3$)amino ou un groupe N-benzyl-N-méthyl-amino,

$R^3$ représente un groupe alkyle en $C_1$-$C_4$ éventuellement substitué par un groupe hydroxyle, et

$R^4$ représente un radical alkyle à chaîne droite, à chaîne ramifiée ou cyclique contenant jusqu'à

6 atomes de carbone,

sous forme de leurs isomères, de leurs mélanges d'isomères, de leurs racémates et de leurs antipodes optiques, ainsi que de leurs sels physiologiquement acceptables, **caractérisé en ce qu'**on fait réagir

[A] des aldéhydes de formule générale II

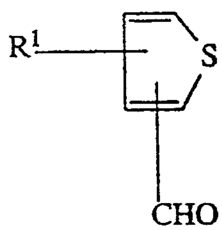

(II)

dans laquelle

$R^1$ a la signification mentionnée,

avec des esters acétiques de formule générale III

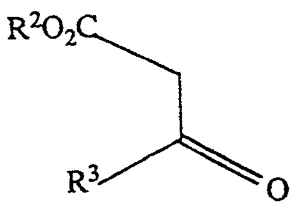

(III)

dans laquelle

$R^2$, $R^3$ ont la signification mentionnée ci-dessus,

avec des énamines de formule générale IV

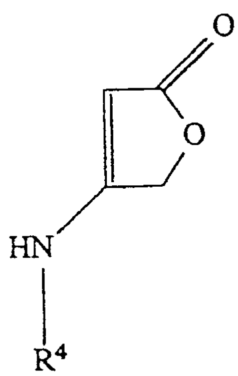

(IV)

dans laquelle

$R^4$ a la signification mentionnée,

dans des solvants inertes, ou lorsqu'on fait réagir

[B] des composés ylidène de formule générale V

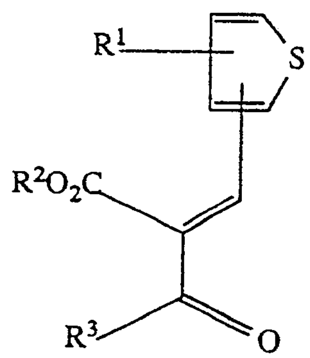

(V)

dans laquelle

$R^1$, $R^2$, $R^3$ ont la signification mentionnée,

avec des énamines de formule IV dans des solvants inertes

ou encore lorsqu'on traite avec des bases

[C] des dihydropyridine-lactones répondant à la formule générale VI

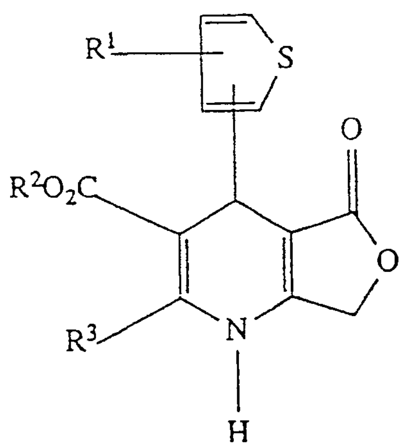

(VI)

dans laquelle

$R^1$ à $R^3$ ont les significations mentionnées,

dans des solvants inertes et ensuite, on alkyle avec des composés de formule générale VII

$R^4$-X (VII)

dans laquelle

$R^4$ a la signification mentionnée et

X représente un atome d'halogène, de préférence un atome de chlore, un atome de brome ou un atome d'iode, ou encore un groupe diazoïque ou un groupe de formule $-O-SO_2R^5$

où

$R^5$ a la signification mentionnée pour $R^4$ ou bien représente un groupe phényle ou un groupe tolyle.

2. Procédé selon la revendication 1, **caractérisé en ce qu'**on effectue la réaction conformément aux variantes de procédé A et B, à des températures de 10 à 200 °C.

3. Procédé selon la revendication 1, **caractérisé en ce qu'**on effectue la réaction conformément à la variante de procédé C, à des températures de -80 à 200 °C.

4. Procédé pour la préparation de médicaments, **caractérisé en ce qu**'on mélange au moins une 4-thiényl-dihydropyridine selon la formule I de la revendication 1, avec des substances de support et auxiliaires habituelles.